# EUROPEAN PATENT SPECIFICATION

(11) **EP 1 401 334 B1**
(45) Date of publication and mention of the grant of the patent: **14.12.2005**
(21) Application number: 02771668.7
(22) Date of filing: 20.05.2002
(51) Int. Cl.: A61B 10/00, A61F 5/455

(54) **A URINE COLLECTION DEVICE**
URINSAMMELVORRICHTUNG
DISPOSITIF DE COLLECTE D'URINE

(30) Priority: 18.05.2001 GB 0112179
(43) Date of publication of application: 31.03.2004
(73) Proprietor: Funnelly Enough Limited, Lowestoft, Suffolk NR32 3LN (GB)
(72) Inventor: FORTE, Vincent, John, Charles, Lowestoft, Suffolk NR33 7AZ (GB)
(74) Representative: Makovski, Priscilla Mary
(86) International application number: PCT/GB2002/002180
(87) International publication number: WO 2002/094104

(56) References cited:
- FR-A- 2 742 981
- GB-A- 2 247 626
- US-A- 4 626 249
- US-A- 4 681 573
- US-A- 4 937 890
- US-A- 5 457 823

## Description

### Field of the Invention

The present invention relates to a device to assist in the collection of urine from a patient. In particular, the device is designed to be useable with one hand and to be disposed of after use.

### Background to the Invention

Urine sampling is an important diagnostic method for detecting a wide range of conditions and illnesses. Analysis of the presence and concentration of chemicals in a urine specimen can, for example, show if a person is pregnant, diabetic, has a kidney dysfunction, has a urinary tract infection etc.

Normally, the specimen is produced by the patient directly into a suitable sterile tube which can then be taken away for analysis. Tubes which are normally used however, have only a narrow neck opening through which to pass the specimen into the tube and to deliver the specimen accurately, without mess, can present difficulties. The difficulties are particularly acute where the patient is female, due to the problem of correctly locating the tube to receive the sample.

A number of devices have been devised to assist people in delivering a urine specimen. Patent publication No's EP 009980 and GB 1497777 disclose two such devices, but these are relatively complex as they are particularly concerned with a collection of an aseptic sample.

US 5 457 823 discloses a urine specimen collector comprising a funnel-like bag having a tong-like member to open the device for use.

The present invention seeks to alleviate the problems of the above disclosed devices, and to provide an easy to use, disposable device to direct urine voided by a female into a sample tube.

### Summary of the Invention

According to the invention there is provided a urine collection device comprising:
a collection means to collect urine voided by the user;
an outlet, integral with the collection means, to direct the urine out of the collection means;
the collection means and outlet being operably moveable between a storage configuration and an expanded in-use configuration, the device including one or more resilient means, so biased to cause the device to move from the storage to the expanded configuration.

The device is thus removed from storage, the resilient means expanding the device directly into a useable configuration with the minimum of inconvenience to the user. The device can be used to collect voided urine, advantageously mid-stream, and direct the urine into a sample tube. The sample tube typically has an opening, of industry standard size, through which the urine must pass.

The device is preferably symmetric in at least the longitudinal plane, to enable the device to be used correctly in more than one orientation and so reduce the possibilities of incorrect use.

Preferably, the device is flat when in its storage configuration. This allows a large number of devices to be stored in a relatively small volume.

The resilient means preferably comprises one or more strips, formed from a resilient material, located on the collection means and/or the outlet means. The strips provide the necessary force to move the collection means from its storage configuration to its in-use configuration with minimum of space uptake and weight. Optionally, the resilient means is an elastic band mounted on the collection means, the elastic band being under tension when the device is in the flat configuration, the tension being releasable to cause the device to open to its in-use configuration. Alternatively, the device can be formed from a material having resilient properties, such as a semi-rigid plastics material or a biodegradable paper.

The collection means is conveniently a funnel to collect and channel urine to the outlet means.

The device preferably includes one or more tabs by which the device for positioning the device so as to reduce the chance of the user accidentally getting urine on their hands. The device can also include a glove portion to cover the user's hand during use, again preventing contamination.

Diagnostic strips can also be included on the surfaces of the device which, in use, come into contact with the urine. The strips can provide information on, for example, the presence of sugar or protein in the urine and so aid diagnosis.

The outlet optionally includes a retention means to hold the device in contact with a sample tube. The outlet means overflow outlet advantageously has a square cross-section to facilitate storage of the device in a flat configuration. Preferably, the outlet is a spout which projects from the body of the collection means. The retention means hinders a sample tube from falling away from the device during use.

Advantageously, the retention means comprises a washer to engage a sample tube and provide a seal to prevent running onto the collection means. Optionally, an adhesive is included to retain a sample tube in position.

The device conveniently includes a further outlet means to direct excess urine out of the device, into for example a toilet, and preventing the overflow of urine from the device onto the user or the floor.

The device is conveniently supplied in a carrier, to minimise damage to and contamination of the device before use and maintain the device in an aseptic condition.

A removable securing tab is preferably included to secure the device in a flat configuration until the device is required.

The device optionally includes a sample tube, removably attached to the outlet means.

### Brief Description of the Drawings

The invention will now be described with reference to the accompanying drawings which show by way of example only, two embodiments of a female urine collection device. In the drawings:
Figure 1 is a side view of a first embodiment of a device in a flattened configuration;
Figure 2 is a side view of a device in an expanded configuration;
Figure 3 is a further side view of the device of Figure 2;
Figure 4 is a plan view of the device of Figure 2;
Figure 5 is a perspective view of a second embodiment of a device in an expedited configuration; and
Figure 6 is a side view of the device of Figure 5.

### Detailed Description of the Invention

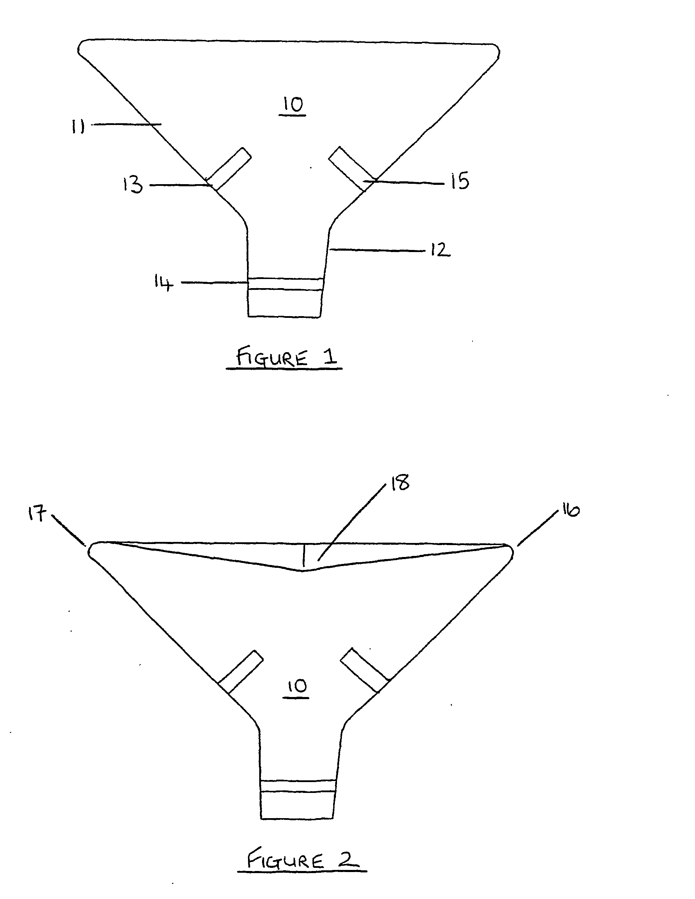

Figure 1 shows a device 10 in a flat configuration, as supplied to the user. The device 10 comprises a funnel portion 11, the funnel portion 11 leading at its narrow end to an outlet 12. The device 10 includes resilient strips 13,14,15, two of which are located on the funnel portion 11, and the third around the outlet 12.

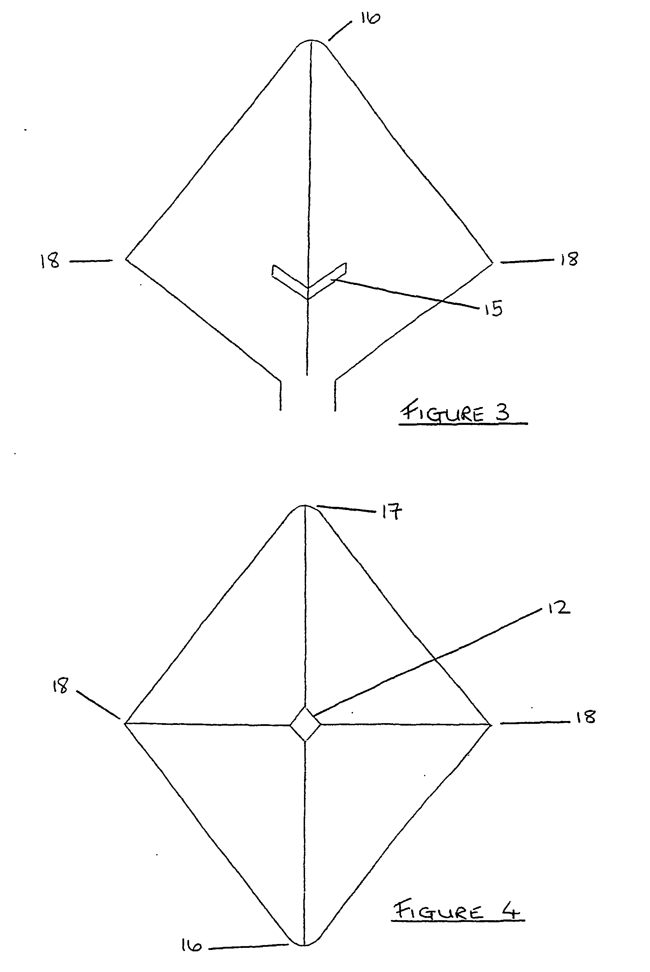

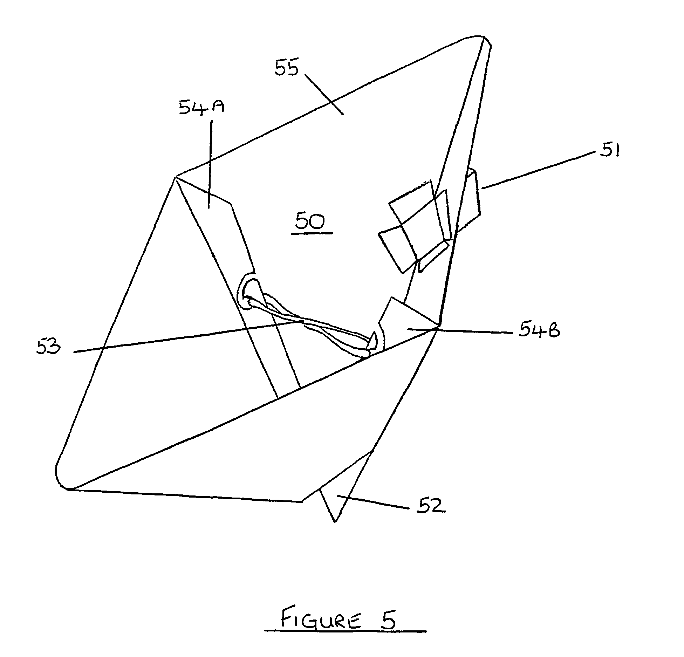

Figures 2 to 4 show the device of Figure 1 in its expanded configuration, prior to use. As shown in Figure 2, the expansion of the device 10, causes the two ends 16,17 of the funnel portion 11 to move upwardly, relative to the centre portion 18, which portion 18 undergoes a slight downward motion. The centre portions 18 (as shown more clearly in Figures 3 and 4) also move away from each other.

The device 10 in the configuration shown in Figures 2 to 4 is well suited to fit closely around the contours of a female body, around the vagina. The outlet 12 is also in a suitable configuration to fit inside a sample tube.

In use, the user removes the device 10 from the packaging in which it is supplied. The resilient members 13,14,15 are biased to open the device to an expanded configuration and thus cause the device 10 to expand from the flat configuration in which it is supplied to the expanded configuration. A sample tube is then passed over the free end of the outlet 12 and held in place during use, either by friction between the material from which the device is made and the sample tube or by the user. The user pushes the funnel portion 11 to ensure that the device 10 is fully engaged with the sample tube, the pushing action also ensuring that the device 10 is fully expanded to its in-use configuration. The user positions the device 10 such that the funnel portion 11 is in the proper position for receiving voided urine.

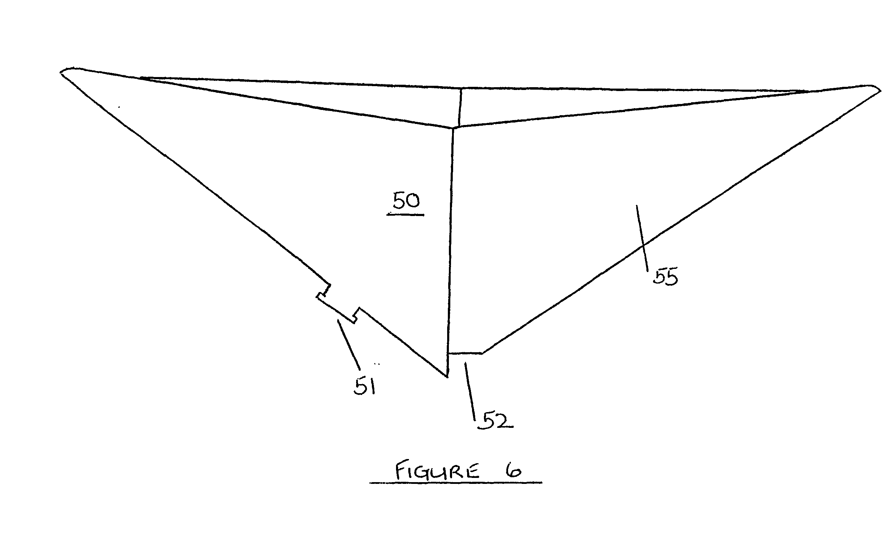

Figures 5 and 6 show a second embodiment of a device 50. The device 50 has two outlets from which urine voided into the device 50 can flow. Firstly, a spout 51 is substantially square in cross-section. The square cross-section not only facilitates the folding of the device 50 into a flat configuration, but also provides a better seal with many sample bottles, particularly those which are routinely used at present. The spout 51, in-use, receives a sample tube to collect urine. To achieve a better seal between the sample tube and the device, a washer formed of a spongy material is located around the base of the spout 51. The second outlet 52 ensures the device does not overflow should the flow of urine be too rapid or the sample bottle fill up. An advantage of the spout 51 being on the side of funnel is that as the sample bottle is filled up by the user, it is lying on its side. Once the user is finished, the bottle can be held upright and the level of the liquid in the bottle then automatically falls below the bottle's brim.

An elastic band 53 causes the device 50 to open from its flat storage configuration to its open configuration. The elastic band 53 is passed around housings 54A, 54B on the inside of the funnel portion 55 of the device 50. In the storage configuration, the elastic band 53 is stretched and under tension. When the force maintained in the device 50 in this storage configuration is removed, the tension in the elastic band 53 causes the housings 54A, 54B to be pulled towards each other which brings the device 50 into its in-use configuration.

To hold the device 50 in its storage configuration, a paper clip may simply be used. Alternatively, an adhesive can be employed to hold portions of the walls of the device 50 together.

The user then passes urine, the urine being collected in the sample tube. Once the sample tube has been removed, the device can be hygienically disposed of

The device is made from a wax-coated paper, the wax coating not permitting passage of the urine and thus preventing the paper from becoming soggy and unusable. The resilient strips are formed from a plastics material such as a polyethylene or polypropylene.

The device can include a number of other features which do not depart from the scope of the invention. For example, the device can itself be formed from a resilient material whose natural configuration is that of the expanded device. Upon removal of the device from its packaging material, the device expands to its in-use configuration. The elastic band can be located around the outside of the device to pull the device into its in-use configuration.

Although the embodiment of the device described in the drawings is symmetric about the plane of the device's longitudinal axis, the device can be formed asymmetric when required, without impairment of the function. In particular, an asymmetric device can assist in preventing urine from running out of the device when the device must be held at a non-horizontal angle during use.

One or more tabs can be included to assist the user either to remove the device from the packaging material in which the device is supplied. Additionally or alternatively, the tab can be used to hold the device during use. A glove can be present on the device, into which glove the user inserts their hand to grip the device. The user's hand is thus protected from splashing during use of the device.

The outlet of the device can include an adhesive on one or more of its surfaces to hold a sample tube more securely in position. The outlet itself would typically be substantially cylindrical, forming a tube-like structure. Such an outlet tube can be slightly tapered towards its free end to more easily pass into a sample tube. Alternatively, the free end can be tapered outwardly to pass over the sample tube. An "O"-seal formed from a natural or synthetic rubber ensures that a good seal is provided between the device and the sample tube, to minimise the possibility of urine running out of the device and over the user or the floor.

Diagnostic strips or areas can be included on the surfaces of the device which come into contact with the voided urine. For example, chemicals can be present in the strips or areas, the chemicals undergoing a chromatic change in the presence of sugar or protein in the urine. The device can therefore be used to give a quick indication to a medical practitioner of the state of health of the user.

In addition to the primary outlet to the sample tube, the device can include one or more further outlets to direct excess urine away from the user, and when directed accordingly, into, for example a toilet.

The device would normally be provided to the user in a flat configuration, inside a carrier such as an envelope. The carrier maintains the device in a clean and, where required, aseptic condition.

It will of course be understood that the invention is not limited to the specific details described herein, which are given by way of example only, and that various modifications and alterations are possible within the scope of the invention as defined by the claims.

## Claims

1. A urine collection device (50) comprising:
a collection means (55) to collect urine voided by a user;
an outlet means (51), integral with the collection means (55), to direct the urine out of the collection means (55);
the collection means (55) and outlet means (51) being operably moveable between a storage configuration and an expanded in-use configuration, the device (50) including one or more resilient means (53), so biased to cause the device (50) to move from the storage to the expanded configuration.

2. A urine collection device according to Claim 1, wherein the device is symmetric at least along a central longitudinal plane.

3. A device according to either Claim 1 or Claim 2, wherein the device is flat when in its storage configuration.

4. A urine collection device according to any preceding claim, wherein the or each resilient means (53) comprises one or more resilient strips located on one or both of the collection means and the outlet means.

5. A urine collection device according to any preceding claim, wherein the resilient means (53) includes an elastic band mounted on the collection means (55).

6. A urine collection device according to any one of the preceding claims, wherein the device is formed substantially wholly of a self-supporting resilient material.

7. A female urine collection device according to Claim 6, wherein the self-supporting resilient material is a semi-rigid plastics material.

8. A urine collection device according to Claim 6, wherein the resilient material is biodegradable

9. A urine collection device according to any one of the preceding claims, wherein the collection means is funnel shaped to collect and channel urine to the outlet means.

10. A urine collection device according to any one of the preceding claims, which includes one or more tabs for positioning the device in-use so as to reduce the risk of a user or individual handling the device coming into direct contact with the urine.

11. A urine collection device according to any one of the preceding claims, wherein the device includes a glove portion to cover the user's hand during use.

12. A urine collection device according to any one of the preceding claims, wherein the device includes diagnostic strips on the surfaces of the device which, in use, come into contact with urine.

13. A urine collection device according to any one of the preceding claims, wherein the outlet means includes an adhesive strip to hold the device in contact with a sample tube.

14. A urine collection device according to any preceding claim, wherein the outlet means is a spout.

15. A urine collection device according to Claim 14, wherein the spout has, in-use, a square or rectangular cross-section.

16. A urine collection device according to any one of the preceding claims, which includes a sample tube, removably attached to the outlet means.

17. A urine collection device according to any one of the preceding claims, which includes an overflow means (52) to direct excess urine out of the device and preventing the overflow of urine from the device.

18. A urine collection device according to any one of the preceding claims, wherein the device is supplied in a flat folded configuration and held in such configuration by packaging material.

## Patentansprüche

1. Urinsammelbehälter (50), umfassend:
eine Sammeleinrichtung (55) zum Sammeln des von einem Benutzer gelassenen Urins;
eine Auslaßeinrichtung (51), die mit dem Sammelbehälter (55) integral ausgebildet ist, um das Urin aus der Sammeleinrichtung (55) herauszuleiten;
wobei die Sammeleinrichtung (55) und Auslaßeinrichtung (51) betriebsmäßig zwischen einer Lagerungskonfiguration und einer ausgedehnten Benutzungskonfiguration beweglich sind, wobei die Vorrichtung (50) ein oder mehrere federnde Einrichtungen (53) besitzt, die derart vorgespannt sind, daß sie die Vorrichtung (50) veranlassen, sich von der Lagerungskonfiguration in die ausgedehnte Konfiguration zu bewegen.

2. Urinsammelvorrichtung gemäß Anspruch 1, wobei die Vorrichtung zumindest entlang einer zentralen Längsebene symmetrisch ist.

3. Vorrichtung gemäß entweder Anspruch 1 oder Anspruch 2, wobei die Vorrichtung in ihrer Lagerungskonfiguration flach ist.

4. Urinsammelvorrichtung gemäß einem der voranstehenden Ansprüche, wobei die oder jede federnde Einrichtung (53) ein oder mehrere federnde Streifen umfaßt, die auf einer oder beiden der Sammeleinrichtung und der Auslaßeinrichtung angeordnet sind.

5. Urinsammelvorrichtung gemäß einem der voranstehenden Ansprüche, wobei die federnde Einrichtung (53) ein an der Sammeleinrichtung (55) befestigtes elastisches Band aufweist.

6. Urinsammelvorrichtung gemäß einem der voranstehenden Ansprüche, wobei die Vorrichtung im wesentlichen vollständig aus einem selbsttragenden federnden Material gebildet ist.

7. Urinsammelvorrichtung für Frauen gemäß Anspruch 6, wobei das selbsttragende federnde Material ein halbsteifes Kunststoffmaterial ist.

8. Urinsammelvorrichtung gemäß Anspruch 6, wobei das federnde Material biologisch abbaubar ist.

9. Urinsammelvorrichtung gemäß einem der voranstehenden Ansprüche, wobei die Sammeleinrichtung trichterförmig ist, um Urin zu sammeln und zu der Auslaßeinrichtung zu kanalisieren.

10. Urinsammelvorrichtung gemäß einem der voranstehenden Ansprüche, welche eine oder mehrere Laschen zur Positionierung der Vorrichtung im Gebrauch aufweist, um das Risiko eines die Vorrichtung handhabenden Benutzers oder Individuums zu reduzieren, mit dem Urin in direkten Kontakt zu kommen.

11. Urinsammelvorrichtung gemäß einem der voranstehenden Ansprüche, wobei die Vorrichtung einen Handschuhabschnitt aufweist, um während der Benutzung die Hand des Benutzers abzudecken.

12. Urinsammelvorrichtung gemäß einem der voranstehenden Ansprüche, wobei die Vorrichtung Diagnosestreifen auf den Oberflächen der Vorrichtung aufweist, welche in Gebrauch mit dem Urin in Kontakt kommen.

13. Urinsammelvorrichtung gemäß einem der voranstehenden Ansprüche, wobei die Auslaßeinrichtung einen Klebestreifen aufweist, um die Vorrichtung in Kontakt mit einem Proberöhrchen zu halten.

14. Urinsammelvorrichtung gemäß einem der voranstehenden Ansprüche, wobei die Auslaßeinrichtung ein Ausgießer ist.

15. Urinsammelvorrichtung gemäß Anspruch 14, wobei der Ausgießer in Gebrauch einen quadratischen oder rechteckigen Querschnitt besitzt.

16. Urinsammelvorrichtung gemäß einem der voranstehenden Ansprüche, welche ein Proberöhrchen aufweist, das lösbar an der Auslaßeinrichtung befestigt ist.

17. Urinsammelvorrichtung gemäß einem der voranstehenden Ansprüche, welches eine Überlaufeinrichtung (52) aufweist, um überschüssigen Urin aus der Vorrichtung zu leiten und den Überlauf von Urin aus der Vorrichtung zu verhindern.

18. Urinsammelvorrichtung gemäß einem der voranstehenden Ansprüche, wobei die Vorrichtung in einer flach gefalteten Konfiguration zur Verfügung gestellt und in dieser Konfiguration durch Verpackungsmaterial gehalten ist.

## Revendications

1. Dispositif de collecte d'urine (50) comprenant :
des moyens de collecte (55) destinés à collecter l'urine émise par un utilisateur ;
des moyens d'orifice de sortie (51), d'un seul tenant avec les moyens de collecte (55), destinés à diriger l'urine hors des moyens de collecte (55) ;
les moyens de collecte (55) et les moyens d'orifice de sortie (51) étant fonctionnellement déplaçables entre une configuration de stockage et une configuration d'utilisation déployée, le dispositif (50) comprenant un ou plusieurs moyens résilients (53), sollicités de telle sorte qu'ils amènent le dispositif (50) à se déplacer de la configuration de stockage à la configuration déployée.

2. Dispositif de collecte d'urine selon la revendication 1, dans lequel le dispositif est symétrique au moins le long d'un plan central longitudinal.

3. Dispositif selon l'une ou l'autre de la revendication 1 ou de la revendication 2, dans lequel le dispositif est plat lorsqu'il se trouve dans sa configuration de stockage.

4. Dispositif de collecte d'urine selon l'une quelconque des revendications précédentes, dans lequel le ou chacun des moyens résilients (53) comprend une ou plusieurs bandes résilientes situées sur les moyens de collecte ou sur les moyens d'orifice de sortie ou sur les deux.

5. Dispositif de collecte d'urine selon l'une quelconque des revendications précédentes, dans lequel les moyens résilients (53) comprennent une bande élastique montée sur les moyens de collecte (55).

6. Dispositif de collecte d'urine selon l'une quelconque des revendications précédentes, dans lequel le dispositif est formé sensiblement totalement par un matériau résilient autoporteur.

7. Dispositif de collecte d'urine pour femme selon la revendication 6, dans lequel le matériau résilient autoporteur est un matériau de plastique semi-rigide.

8. Dispositif de collecte d'urine selon la revendication 6, dans lequel le matériau résilient est biodégradable.

9. Dispositif de collecte d'urine selon l'une quelconque des revendications précédentes, dans lequel les moyens de collecte sont en forme d'entonnoir pour collecter et canaliser l'urine vers les moyens d'orifice de sortie.

10. Dispositif de collecte d'urine selon l'une quelconque des revendications précédentes, qui comprend une ou plusieurs languettes destinées à positionner le dispositif durant l'utilisation de manière à réduire le risque qu'un utilisateur ou un individu manipulant le dispositif soit au contact direct de l'urine.

11. Dispositif de collecte d'urine selon l'une quelconque des revendications précédentes, dans lequel le dispositif comprend une partie de gant destinée à couvrir la main de l'utilisateur durant l'utilisation.

12. Dispositif de collecte d'urine selon l'une quelconque des revendications précédentes, dans lequel le dispositif comprend des bandelettes diagnostiques sur les surfaces du dispositif qui, durant l'utilisation, sont en contact avec l'urine.

13. Dispositif de collecte d'urine selon l'une quelconque des revendications précédentes, dans lequel les moyens d'orifice de sortie comprennent une bande adhésive destinée à maintenir le dispositif en contact avec un tube échantillon.

14. Dispositif de collecte d'urine selon l'une quelconque des revendications précédentes, dans lequel les moyens d'orifice de sortie sont un bec verseur.

15. Dispositif de collecte d'urine selon la revendication 14, dans lequel le bec verseur a, durant l'utilisation, une section transversale carrée ou rectangulaire.

16. Dispositif de collecte d'urine selon l'une quelconque des revendications précédentes, qui comprend un tube échantillon, fixé de manière détachable aux moyens d'orifice de sortie.

17. Dispositif de collecte d'urine selon l'une quelconque des revendications précédentes, qui comprend des moyens de trop plein (52) destinés à diriger un excès d'urine hors du dispositif et à empêcher le débordement d'urine du dispositif.

18. Dispositif de collecte d'urine selon l'une quelconque des revendications précédentes, dans lequel le dispositif est fourni dans une configuration plate et pliée et est maintenu dans cette configuration par un matériau d'emballage.
